# EUROPEAN PATENT APPLICATION

(11) **EP 4 793 960 A1**
(43) Date of publication of application: **19.08.2026**
(21) Application number: 25227620.9
(22) Date of filing: 30.12.2025
(51) Int. Cl.: G16H 15/00

(54) **METHOD AND DEVICE FOR GENERATING CRF FORM FOR RARE DISEASE**

(30) Priority: 14.02.2025 CN 202510163036
(71) Applicant: Peking Union Medical College Hospital, Chinese Academy of Medical Sciences & Peking Union Medical College, Beijing 100730 (CN); Digital Health China Technologies Co., Ltd., Beijing 100080 (CN)
(72) Inventor: GUO, Jian, Beijing, 100730 (CN); ZHANG, Shuyang, Beijing, 100730 (CN); JIN, Ye, Beijing, 100730 (CN); ZHANG, Wen, Beijing, 100730 (CN); GONG, Mengchun, Beijing, 100080 (CN); SHI, Wenzhao, Beijing, 100080 (CN); ZHENG, Xihong, Beijing, 100080 (CN); XIA, Peng, Beijing, 100730 (CN); PENG, Linyi, Beijing, 100730 (CN); LIU, Peng, Beijing, 100730 (CN)
(74) Representative: Lapienis, Juozas

(57) **Abstract**

Disclosed are a method and a device for generating a CRF form for a rare disease. When the CRF form is required, a user inputs a keyword of the rare disease through the user terminal, and a medical report database is provided in a server. Since medical reports stored in the medical report database include items required to be filled in a CRF form corresponding to a related disease, such as patient basic information, past medical history, personal physical examination results, medical examination results, imaging examination results, and doctor opinions and suggestions, various items capable of establishing the CRF form can be obtained from the medical reports corresponding to the rare disease. A comparison with medical terms included in the medical lexicon is performed to determine a medical report having a high relevance to the keyword, which facilitates construction of the CRF form related to the current rare disease. Through the above method, the user can obtain a corresponding CRF form only by inputting the keyword, which is beneficial to reducing manual workload.

## Description

### TECHNICAL FIELD

The present disclosure relates to the field of computer technology, and specifically, to a method and a device for generating a CRF form for a rare disease.

### BACKGROUND

The configuration of medical CRFs (case report forms) is usually customized according to the research requirements of investigators, such as clinicians, public health physicians, pharmacists, and health economists, to determine the specific content of the CRF forms. For rare diseases, different disease types or subtypes exhibit both homogeneity and heterogeneity, so the data collection form content required for different disease types or subtypes also differs. Against this background, the customized CRF form lacks sufficient flexibility. When the research content or disease type changes, manual modification of the customized CRF form is required, resulting in a large amount of manual work.

### SUMMARY

In view of this, an embodiment of the present disclosure provides a method and device for generating a CRF form for a rare disease, to provide corresponding CRF forms for different rare diseases, thereby reducing manual workload.

According to a first aspect, an embodiment of the present disclosure provides a method for generating a CRF form for a rare disease. The generating method is applied in a form generation system, the form generation system includes a user terminal and a server, the server is provided with a medical report database and a medical lexicon, and the generating method runs on the server and includes:
in response to a keyword input by a user at the user terminal, performing word frequency statistics on each medical report in the medical report database to determine a candidate word with a highest word frequency in each medical report;
for each medical term in the medical dictionary and each to-be-compared word, constructing a matrix of (m+1)×(n+1) based on a first string length of the medical term and a second string length of the to-be-compared word, where m is the first string length, n is the second string length, and the to-be-compared word includes the keyword and the candidate word;
initializing a first row of elements in the matrix sequentially with integers from 0 to n and a first column of elements in the matrix sequentially with integers from 0 to m; for other matrix elements Tij in the matrix, determining other matrix elements Tij according to a preset rule, where the preset rule includes: if an i-th character of the medical term and a j-th character of the to-be-compared word are identical, setting the matrix element Tij equal to a matrix element in an upper-left matrix cell adjacent to a matrix cell where the matrix element Tij is located; if an i-th character of the medical term and a j-th character of the to-be-compared word are not identical, setting the matrix element Tij equal to a sum of a minimum matrix element in upper-left, top, and left matrix cells adjacent to a matrix cell where the matrix element Tij is located and a value 1;
after determining matrix elements in matrix cells of the matrix, calculating a similarity between the medical term and the to-be-compared word using the following formula: $S = 1 - B / A ;$
where B is a matrix element in a lower-right matrix cell of the matrix, and A is the greater of m and n;
after obtaining a first similarity between each candidate word and each medical term and a second similarity between the keyword and each medical term, determining a target medical term corresponding to the first similarity between the candidate word and the medical term that has a minimum difference from a target similarity, where the target medical term is a medical term corresponding to a maximum first similarity for each candidate word, and the target similarity is a maximum second similarity; and
constructing a CRF form based on hierarchical headings in a target medical report corresponding to the target medical term and words matching numerical values in text content corresponding to the hierarchical headings.

Optionally, constructing the CRF form based on the hierarchical headings in the target medical report corresponding to the target medical term and the words matching the numerical values in the text content corresponding to the hierarchical headings includes:
after obtaining the hierarchical headings in the target medical report, performing tokenization on the hierarchical headings to obtain candidate tokens included in the hierarchical headings;
for each candidate token, after determining part-of-speech of the candidate token, determining a distribution ratio of the part-of-speech in various parts-of-speech in the medical field as a weight of the candidate token;
calculating a similarity between the candidate token and a preset medical field term using the following formula: $S = \frac{K . L}{\left‖K\right‖ . \left‖L\right‖} d ;$
where K is a first word vector corresponding to the candidate token, L is a second word vector corresponding to the preset medical field term, **||K||** is a magnitude of the first word vector, **||L||** is a magnitude of the second word vector, and d is the weight;
processing text content corresponding to each hierarchical heading to obtain text tokens and a dependency relationship between the text tokens;
determining candidate text tokens that have a dependency relationship with text tokens whose part-of-speech is numeral based on the parts-of-speech of the text tokens;
selecting text tokens having a specified part-of-speech from the candidate text tokens as target text tokens; and
taking candidate tokens with a similarity higher than a preset threshold as parent items, and for each parent item, constructing the CRF form by taking a target text token included in a text content under a heading corresponding to the parent item as a child item.

Optionally, the generating method further includes:
displaying the CRF form on the user terminal.

Optionally, the user terminal includes a mobile terminal and a fixed terminal.

Optionally, the generating method further includes:
in response to an adding operation of a user, adding an item corresponding to the adding operation at a position specified by the adding operation.

According to a second aspect, an embodiment of the present disclosure provides a device for generating a CRF form for a rare disease. The generating device is applied in a form generation system, the form generation system includes a user terminal and a server, the server is provided with a medical report database and a medical lexicon, and the generating device is configured on the server and includes:
a statistical unit, configured to, in response to a keyword input by a user at the user terminal, perform word frequency statistics on each medical report in the medical report database to determine a candidate word with a highest word frequency in each medical report;
a construction unit, configured to, for each medical term in the medical dictionary and each to-be-compared word, construct a matrix of (m+1)×(n+1) based on a first string length of the medical term and a second string length of the to-be-compared word, where m is the first string length, n is the second string length, and the to-be-compared word includes the keyword and the candidate word;
a first determination unit, configured to initialize a first row of elements in the matrix sequentially with integers from 0 to n and a first column of elements in the matrix sequentially with integers from 0 to m; for other matrix elements Tij in the matrix, determine other matrix elements Tij according to a preset rule, where the preset rule includes: if an i-th character of the medical term and a j-th character of the to-be-compared word are identical, set the matrix element Tij equal to a matrix element in an upper-left matrix cell adjacent to a matrix cell where the matrix element Tij is located; if an i-th character of the medical term and a j-th character of the to-be-compared word are not identical, set the matrix element Tij equal to a sum of a minimum matrix element in upper-left, top, and left matrix cells adjacent to a matrix cell where the matrix element Tij is located and a value 1;
a calculation unit, configured to, after determining matrix elements in matrix cells of the matrix, calculate a similarity between the medical term and the to-be-compared word using the following formula: $S = 1 - B / A ,$
where B is a matrix element in a lower-right matrix cell of the matrix, and A is the greater of m and n;
a second determination unit, after obtaining a first similarity between each candidate word and each medical term and a second similarity between the keyword and each medical term, determine a target medical term corresponding to the first similarity between the candidate word and the medical term that has a minimum difference from a target similarity, where the target medical term is a medical term corresponding to a maximum first similarity for each candidate word, and the target similarity is a maximum second similarity; and
a generation unit, configured to construct a CRF form based on hierarchical headings in a target medical report corresponding to the target medical term and words matching numerical values in text content corresponding to the hierarchical headings.

Optionally, the generation unit being configured to construct the CRF form based on the hierarchical headings in the target medical report corresponding to the target medical term and the words matching the numerical values in the text content corresponding to the hierarchical headings includes:
after obtaining the hierarchical headings in the target medical report, performing tokenization on the hierarchical headings to obtain candidate tokens included in the hierarchical headings;
for each candidate token, after determining part-of-speech of the candidate token, determining a distribution ratio of the part-of-speech in various parts-of-speech in the medical field as a weight of the candidate token;
calculating a similarity between the candidate token and a preset medical field term using the following formula: $S = \frac{K . L}{\left‖K\right‖ . \left‖L\right‖} d ;$
where K is a first word vector corresponding to the candidate token, L is a second word vector corresponding to the preset medical field term, ∥K∥ is a magnitude of the first word vector, ∥L∥ is a magnitude of the second word vector, and d is the weight;
processing text content corresponding to each hierarchical heading to obtain text tokens and a dependency relationship between the text tokens;
determining candidate text tokens that have a dependency relationship with text tokens whose part-of-speech is numeral based on the parts-of-speech of the text tokens;
selecting text tokens having a specified part-of-speech from the candidate text tokens as target text tokens; and
taking candidate tokens with a similarity higher than a preset threshold as parent items, and for each parent item, constructing the CRF form by taking a target text token included in a text content under a heading corresponding to the parent item as a child item.

Optionally, the generating device further includes:
a display unit, configured to display the CRF form on the user terminal.

Optionally, the user terminal includes a mobile terminal and a fixed terminal.

Optionally, the generating device further includes:
an adding unit, configured to, in response to an adding operation of a user, add an item corresponding to the adding operation at a position specified by the adding operation.

The technical solutions provided in the embodiments of the present disclosure have the following beneficial effects:
In the present disclosure, when a CRF form for a rare disease is required, a user can input a keyword of the rare disease through the user terminal, and a medical report database is provided in a server. Since medical reports stored in the medical report database include items required to be filled in a CRF form corresponding to a related disease, such as patient basic information, past medical history, personal physical examination results, medical examination results, imaging examination results, and doctor opinions and suggestions, various items capable of establishing the CRF form can be obtained from the medical reports corresponding to the rare disease. A comparison with medical terms included in the medical lexicon is performed so as to determine a medical report having a high relevance to the keyword, which facilitates construction of the CRF form related to the current rare disease. Through the above method, the user can obtain a corresponding CRF form only by inputting the keyword, which is beneficial to reducing manual workload.

To make the above objectives, features and advantages of the present disclosure comprehensible, preferred embodiments with reference to the accompanying drawings are described in detail below.

### BRIEF DESCRIPTION OF THE DRAWINGS

To more clearly illustrate the technical solutions of the embodiments of the present disclosure, the drawings required in the embodiments will be briefly described below. It should be understood that the following drawings only illustrate some embodiments of the present disclosure and therefore should not be considered as limitations of the scope, and for those of ordinary skill in the art, other related drawings can be obtained according to these drawings without inventive efforts.
FIG. 1 is a flow schematic diagram of a method for generating a CRF form for a rare disease provided in an embodiment of the present disclosure;
FIG. 2 is another flow schematic diagram of a method for generating a CRF form for a rare disease provided in an embodiment of the present disclosure; and
FIG. 3 is a schematic diagram of a structure of a device for generating a CRF form for a rare disease provided in an embodiment of the present disclosure.

### DETAILED DESCRIPTION OF THE EMBODIMENTS

To make the objectives, technical solutions, and advantages of embodiments of the present disclosure clearer, the following clearly and completely describes the technical solutions in embodiments of the present disclosure with reference to the accompanying drawings in embodiments of the present disclosure. It is clear that the described embodiments are merely some rather than all of embodiments of the present disclosure. The assemblies of embodiments of the present disclosure, as generally described and illustrated in the figures herein, can be arranged and designed in a wide variety of different configurations. Therefore, the following detailed descriptions of the embodiments of the present disclosure provided in the drawings are not intended to limit the scope of the claimed disclosure, but merely to represent selected embodiments of the present disclosure. All other embodiments obtained by a person skilled in the art based on the embodiments of the present disclosure without creative efforts shall fall within the protection scope of the present disclosure.

It should be noted in advance that the medical report involved in the present disclosure is an instructive medical document capable of serving as guidance for doctors or medical workers to perform treatment and research related to a rare disease, for example, instructive medical documents that guide how to diagnose a related disease, how to treat a related disease, related precautions, and related disease condition indicators. Specific medical reports stored in the medical report database may be set according to actual requirements, and no specific limitation is made herein.

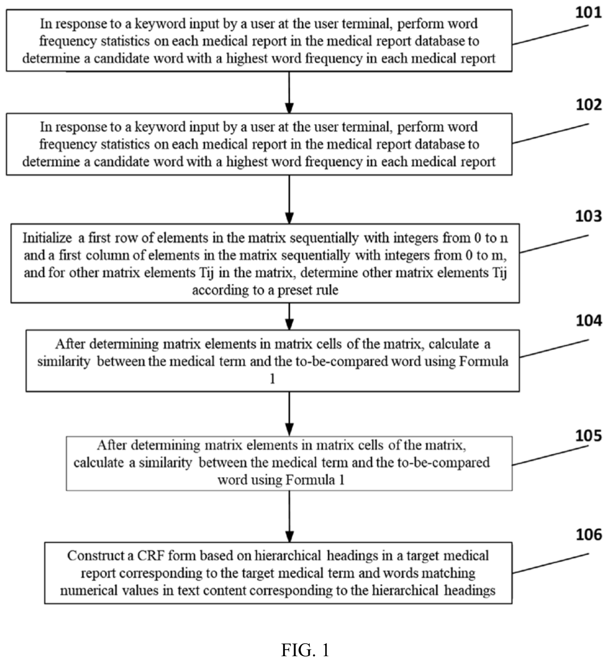

FIG. 1 is a flow schematic diagram of a method for generating a CRF form for a rare disease provided in an embodiment of the present disclosure. The generating method is applied in a form generation system, the form generation system includes a user terminal and a server, the server is provided with a medical report database and a medical lexicon, and the generating method runs on the server. As shown in FIG. 1, the generating method includes the following steps S101 to S106.
S101: In response to a keyword input by a user at the user terminal, word frequency statistics is performed on each medical report in the medical report database to determine a candidate word with a highest word frequency in each medical report.

S102: For each medical term in the medical dictionary and each to-be-compared word, a matrix of (m+1)×(n+1) is constructed based on a first string length of the medical term and a second string length of the to-be-compared word, where m is the first string length, n is the second string length, and the to-be-compared word includes the keyword and the candidate word.

S103: A first row of elements in the matrix are sequentially initialized with integers from 0 to n, and a first column of elements in the matrix are sequentially initialized with integers from 0 to m; for other matrix elements Tij in the matrix, other matrix elements Tij are determined according to a preset rule, where the preset rule includes: if an i-th character of the medical term and a j-th character of the to-be-compared word are identical, the matrix element Tij is set equal to a matrix element in an upper-left matrix cell adjacent to a matrix cell where the matrix element Tij is located; if an i-th character of the medical term and a j-th character of the to-be-compared word are not identical, the matrix element Tij is set equal to a sum of a minimum matrix element in upper-left, top, and left matrix cells adjacent to a matrix cell where the matrix element Tij is located and a value 1.

S104: After determining matrix elements in matrix cells of the matrix, a similarity between the medical term and the to-be-compared word is calculated using the following formula: $S = 1 - B / A ;$ where B is a matrix element in a lower-right matrix cell of the matrix, and A is the greater of m and n.

S105: After a first similarity between each candidate word and each medical term and a second similarity between the keyword and each medical term are obtained, a target medical term corresponding to the first similarity between the candidate word and the medical term that has a minimum difference from a target similarity is determined, where the target medical term is a medical term corresponding to a maximum first similarity for each candidate word, and the target similarity is a maximum second similarity.

S106: A CRF form is constructed based on hierarchical headings in a target medical report corresponding to the target medical term and words matching numerical values in text content corresponding to the hierarchical headings.

Specifically, when a user (including but not limited to a doctor, a medical worker, or a disease researcher) needs to use a CRF form for a rare disease, a keyword related to the rare disease can be inputted in the user terminal, for example, a disease name of the rare disease. After the server obtains the keyword, word frequency statistics are performed on each medical report stored in the medical report database, and a candidate word with the highest word frequency in each medical report is determined. A specific disease type targeted by a corresponding medical report can be determined by selecting the candidate word with the highest word frequency in the medical report. For a disease, differences may exist in descriptions. To determine standard expressions corresponding to each candidate word and the keyword, determination may be performed through steps S102 to S104. Taking «kitten» as a medical term in the medical lexicon and «sitting» as a to-be-compared word as an example, a string length of «kitten» is 6, and a string length of «sitting» is 7. A 7*8 matrix is constructed, the first row of matrix elements of the 7*8 matrix are sequentially initialized to 0-7, and the first column of matrix elements of the 7*8 matrix are sequentially initialized to 0-6. A resulting matrix is as follows:

| | | | | | | | |
|---|---|---|---|---|---|---|---|
| 0 | 1 | 2 | 3 | 4 | 5 | 6 | 7 |
| 1 | | | | | | | |
| 2 | | | | | | | |
| 3 | | | | | | | |
| 4 | | | | | | | |
| 5 | | | | | | | |
| 6 | | | | | | | |

For other matrix elements Tij (a matrix formed by blank matrix cells in the above matrix), sequential determination is performed from left to right and from top to bottom. A preset rule used during determination is as follows: when an i-th character of the medical term and a j-th character of the to-be-compared word are identical, the matrix element Tij is equal to a matrix element in an upper-left matrix cell adjacent to the matrix cell in which the matrix element Tij is located; and when an i-th character of the medical term and a j-th character of the to-be-compared word are not identical, the matrix element Tij is equal to a sum of a minimum matrix element in upper-left, top, and left matrix cells adjacent to a matrix cell where the matrix element Tij is located and a value 1.

Taking a matrix element in a second-row and second-column cell of the above matrix as an example, Tij is T11, namely a first character of the medical term and a first character of the to-be-compared word. The first character of the medical term is k, and the first character of the to-be-compared word is s. A minimum matrix element in upper-left, top, and left matrix cells adjacent to the T11 cell is 0, and a sum of the minimum matrix element and a value 1 is 1. Therefore, the value 1 is used as the matrix element in the T11 cell.

Taking a matrix element in a third-row and third-column cell of the above matrix as another example, Tij is T22, namely a second character of the medical term and a second character of the to-be-compared word. The second character of the medical term is i, and the second character of the to-be-compared word is also i. an upper-left matrix cell adjacent to the T22 cell is T11. Therefore, a matrix element in the T22 cell is 1.

Through the above method, matrix elements in each matrix cell in the above matrix can be obtained. In this case, a matrix element in a lower-right matrix cell in the above matrix can be obtained, and A is 7. A similarity between the medical term «kitten» and the to-be-compared word «sitting» can then be obtained through Formula 1.

Through the first similarity, a correlation between a candidate word and each medical term (namely a standard medical expression) can be determined. Through the second similarity, a correlation between a keyword inputted by a user and each medical term can be determined. When a correlation between a candidate word and the keyword is high, a difference between similarities of the candidate word and the keyword with a same medical term is generally relatively small. Therefore, to determine which candidate word has a higher correlation with the keyword, after the first similarity between each candidate word and each medical term and the second similarity between the keyword and each medical term are obtained, a medical term with the highest similarity to each candidate word (namely a target medical term) can be determined. In this case, each candidate word corresponds to one target medical term. The target medical term is a term having the highest correlation with a corresponding medical report. In addition, the second similarity between the keyword and each medical term can be determined, and a maximum second similarity can be determined from the second similarities. After the target medical term corresponding to each candidate word is obtained, the first similarity between each candidate word and the target medical term (subsequently referred to as a to-be-compared similarity) can be obtained. A difference between the maximum second similarity and each to-be-compared similarity is then calculated, and the target medical term corresponding to a to-be-compared similarity having a minimum difference is determined. The target medical term can be understood as a medical term having the highest correlation with the keyword. Since each candidate word corresponds to one target medical term, after the target medical term is obtained, a candidate word corresponding to the target medical term is determined. A medical report from which the candidate word originates can thereby be determined, so that the medical report can be used as a target medical report. After the target medical report is obtained, a CRF form can be constructed based on hierarchical headings in the target medical report and words matching numerical values in text content corresponding to the hierarchical headings.

Since the target medical report contains instructive content regarding a disease corresponding to a keyword inputted by a user, instructive items can be obtained from hierarchical headings in the target medical report and words matching numerical values in text content corresponding to the hierarchical headings. Therefore, items in the constructed CRF form have a high correlation with the disease corresponding to the keyword, and the items are relatively complete for user utilization. Through the above method, the user can obtain a corresponding CRF form only by inputting the keyword, which is beneficial to reducing manual workload.

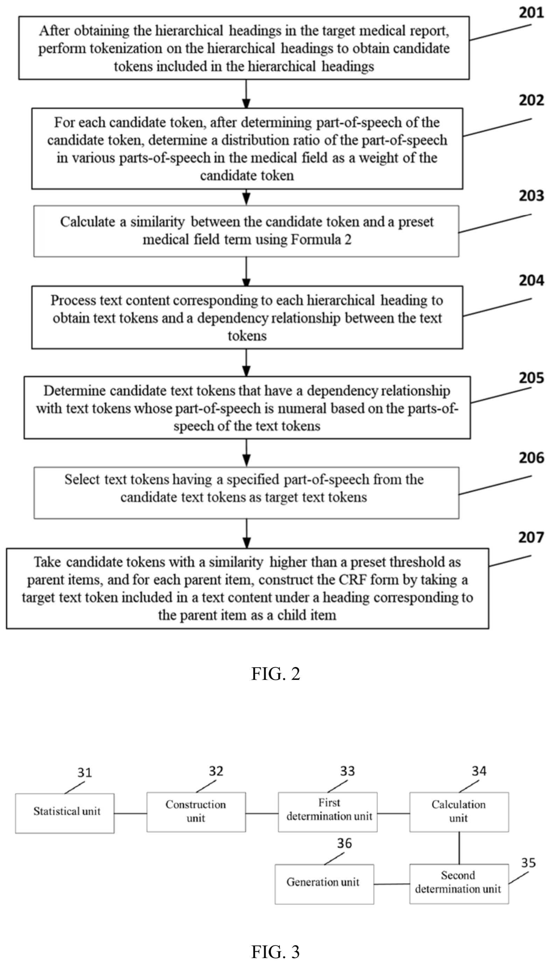

In a possible embodiment, FIG. 2 is a flow schematic diagram of another method for generating a CRF form for a rare disease provided in an embodiment of the present disclosure. As shown in FIG. 2, the step S106 may be implemented by the following steps S201 to S207.
S201: After the hierarchical headings in the target medical report are obtained, tokenization is performed on the hierarchical headings to obtain candidate tokens included in the hierarchical headings.

S202: For each candidate token, after part-of-speech of the candidate token is determined, a distribution ratio of the part-of-speech in various parts-of-speech in the medical field is determined as a weight of the candidate token.

S203: A similarity between the candidate token and a preset medical field term is calculated using the following formula: $S = \frac{K . L}{\left‖K\right‖ . \left‖L\right‖} d ;$ where K is a first word vector corresponding to the candidate token, L is a second word vector corresponding to the preset medical field term, ∥K∥ is a magnitude of the first word vector, ∥L∥ is a magnitude of the second word vector, and d is the weight.

S204: Text content corresponding to each hierarchical heading is processed to obtain text tokens and a dependency relationship between the text tokens.

S205: Candidate text tokens that have a dependency relationship with text tokens whose part-of-speech is numeral is determined based on the parts-of-speech of the text tokens.

S206: Text tokens having a specified part-of-speech from the candidate text tokens are selected as target text tokens.

S207: Candidate tokens with a similarity higher than a preset threshold are taken as parent items, and for each parent item, the CRF form is constructed by taking a target text token included in a text content under a heading corresponding to the parent item as a child item.

Specifically, after the target medical report is obtained, parent items can be constructed based on hierarchical headings, and then sub-items under the parent items can be constructed based on content corresponding to the hierarchical headings, thereby constructing the CRF form. For the hierarchical headings, text tokenization is first performed on each hierarchical heading to obtain candidate tokens included in the hierarchical headings. In the medical field, usage frequencies of different parts-of-speech are different. A higher usage frequency indicates a higher importance of the part-of-speech in the medical field. Therefore, weights can be assigned to corresponding candidate analyses according to a distribution proportion of parts-of-speech in the medical field. For example, if a proportion of nouns in the distribution of parts-of-speech in the medical field is 30%, a distribution weight of the noun part-of-speech is 0.3. After weights corresponding to each candidate token are obtained, a similarity between each candidate token and a preset medical field term is calculated using Formula 2. A higher similarity indicates a higher relevance of the candidate token to the preset medical field.

After the hierarchical headings are obtained, text tokenization is performed on text content corresponding to the hierarchical headings, and dependency relationships among text tokens are determined. The dependency relationships can characterize syntactic dependencies among the tokens. Since a CRF form generally involves filling content related to numerical values, after the text tokens are obtained, numeral tokens related to numerical values in the text tokens are determined. Text tokens having a dependency relationship with the numeral tokens are then used as candidate text tokens, which can serve as sub-items.

Because sub-items generally belong to one or several specific parts-of-speech, for example nouns, after the candidate text tokens are obtained, text tokens of a specified part-of-speech are selected from the candidate text tokens as target text tokens. The candidate tokens having a similarity higher than a preset threshold are used as parent items. For each parent item, the target text tokens included in text content corresponding to a heading of the parent item are used as sub-items, thereby constructing the CRF form. As a result, node relationships expressed by the constructed CRF form correspond to a directory structure in which, for each parent item, target text tokens included in text content corresponding to a heading of the parent item are used as sub-items.

It should be noted that different lookup words and preset medical field terms corresponding to the lookup words can be preset in advance. After the candidate tokens are obtained, a similarity between each candidate token and each lookup word is determined using cosine similarity. A lookup word having the highest similarity is used as a target lookup word of the candidate token, and a preset medical field term corresponding to the target lookup word is used as a preset medical field term of the candidate token. Alternatively, a plurality of preset medical field terms may be preset according to experience. How to obtain the preset medical field terms can be set according to actual requirements, and no specific limitation is made herein.

In a possible embodiment, after the CRF form is obtained, the CRF form is displayed on the user terminal for user viewing.

In a possible embodiment, the user terminal includes a mobile terminal and a fixed terminal.

In a possible embodiment, after the CRF form is obtained, in response to an addition operation by a user, an item corresponding to the addition operation is added to a position designated by the addition operation so as to meet specific requirements of the user.

FIG. 3 is a schematic diagram of a structure of a device for generating a CRF form for a rare disease provided in an embodiment of the present disclosure. The generating device is applied in a form generation system, the form generation system includes a user terminal and a server, the server is provided with a medical report database and a medical lexicon, and the generating device is configured on the server. As shown in FIG. 3, the generating device includes:
a statistical unit 31, configured to, in response to a keyword input by a user at the user terminal, perform word frequency statistics on each medical report in the medical report database to determine a candidate word with a highest word frequency in each medical report;
a construction unit 32, configured to, for each medical term in the medical dictionary and each to-be-compared word, construct a matrix of (m+1)×(n+1) based on a first string length of the medical term and a second string length of the to-be-compared word, where m is the first string length, n is the second string length, and the to-be-compared word includes the keyword and the candidate word;
a first determination unit 33, configured to initialize a first row of elements in the matrix sequentially with integers from 0 to n and a first column of elements in the matrix sequentially with integers from 0 to m; for other matrix elements Tij in the matrix, determine other matrix elements Tij according to a preset rule, where the preset rule includes: if an i-th character of the medical term and a j-th character of the to-be-compared word are identical, set the matrix element Tij equal to a matrix element in an upper-left matrix cell adjacent to a matrix cell where the matrix element Tij is located; if an i-th character of the medical term and a j-th character of the to-be-compared word are not identical, set the matrix element Tij equal to a sum of a minimum matrix element in upper-left, top, and left matrix cells adjacent to a matrix cell where the matrix element Tij is located and a value 1;
a calculation unit 34, configured to, after determining matrix elements in matrix cells of the matrix, calculate a similarity between the medical term and the to-be-compared word using the following formula: $S = 1 - B / A ;$
where B is a matrix element in a lower-right matrix cell of the matrix, and A is the greater of m and n;
a second determination unit 35, after obtaining a first similarity between each candidate word and each medical term and a second similarity between the keyword and each medical term, determine a target medical term corresponding to the first similarity between the candidate word and the medical term that has a minimum difference from a target similarity, where the target medical term is a medical term corresponding to a maximum first similarity for each candidate word, and the target similarity is a maximum second similarity; and
a generation unit 36, configured to construct a CRF form based on hierarchical headings in a target medical report corresponding to the target medical term and words matching numerical values in text content corresponding to the hierarchical headings.

In a possible embodiment, the generation unit being configured to construct the CRF form based on the hierarchical headings in the target medical report corresponding to the target medical term and the words matching the numerical values in the text content corresponding to the hierarchical headings includes:
after obtaining the hierarchical headings in the target medical report, performing tokenization on the hierarchical headings to obtain candidate tokens included in the hierarchical headings;
for each candidate token, after determining part-of-speech of the candidate token, determining a distribution ratio of the part-of-speech in various parts-of-speech in the medical field as a weight of the candidate token;
calculating a similarity between the candidate token and a preset medical field term using the following formula: $S = \frac{K . L}{\left‖K\right‖ . \left‖L\right‖} d ,$
where K is a first word vector corresponding to the candidate token, L is a second word vector corresponding to the preset medical field term, ||K|| is a magnitude of the first word vector, ∥L∥ is a magnitude of the second word vector, and d is the weight;
processing text content corresponding to each hierarchical heading to obtain text tokens and a dependency relationship between the text tokens;
determining candidate text tokens that have a dependency relationship with text tokens whose part-of-speech is numeral based on the parts-of-speech of the text tokens;
selecting text tokens having a specified part-of-speech from the candidate text tokens as target text tokens; and
taking candidate tokens with a similarity higher than a preset threshold as parent items, and for each parent item, constructing the CRF form by taking a target text token included in a text content under a heading corresponding to the parent item as a child item.

In a possible embodiment, the generating device further includes:
a display unit, configured to display the CRF form on the user terminal.

In a possible embodiment, the user terminal includes a mobile terminal and a fixed terminal.

In a possible embodiment, the generating device further includes:
an adding unit, configured to, in response to an adding operation of a user, add an item corresponding to the adding operation at a position specified by the adding operation.

For the descriptions of the related principle of FIG. 3, reference may be made to the descriptions of the related contents of FIGS. 1-2, and details are not repeated herein.

In the embodiments provided in the present disclosure, it should be understood that the disclosed device and method may be implemented in another manner. The described device embodiments are merely examples. For example, the division of units is merely logical function division and may be other division in actual implementation. For example, a plurality of units or components may be combined or integrated into another system, or some features may be ignored or may not be performed. In addition, the displayed or discussed mutual couplings or direct couplings or communications connections may be implemented by using some communication interfaces. The indirect couplings or communications connections between apparatuses or units may be implemented in electrical, mechanical, or other forms.

The units described as separate parts may or may not be physically separate. Parts displayed as units may or may not be physical units, to be specific, may be located in one position, or may be distributed on a plurality of network units. Some or all of the units may be selected based on actual requirements to achieve the objectives of the solutions of embodiments.

In addition, each functional unit in the embodiments provided in the present disclosure can be integrated into one processing unit, or each unit can exist physically independently, or two or more units can be integrated into one unit.

When the functions are implemented in the form of software functional units and sold or used as independent products, the functions can be stored in a computer-readable storage medium. Based on such an understanding, the technical solutions of the present disclosure essentially or parts contributing to the prior art or some of the technical solutions may be embodied in a form of a software product. The computer software product is stored in a storage medium, and includes several instructions for instructing a computer device (which can be a personal computer, a server, a network device, or the like) to perform all or some of the steps of the methods described in the embodiments of the present disclosure. The foregoing storage medium includes any medium that can store program code, such as a USB flash drive, a removable hard disk, a read-only memory (ROM), a random access memory (RAM), a magnetic disk, or an optical disc.

It should be noted that similar reference numerals and letters indicate similar items in the following drawings, and therefore, once an item is defined in one of the drawings, no further definition or explanation is required in the following drawings. In addition, the terms «first», «second», «third», and the like are merely intended for differentiated description, and should not be construed as an indication or an implication of relative importance.

Finally, it should be noted that the foregoing embodiments are only specific implementations of the present disclosure, which are merely intended to describe the technical solutions of the present disclosure, but are not to limit the present disclosure, and the protection scope of the present disclosure is not limited thereto. Although the present disclosure is described in detail with reference to the foregoing embodiments, those of ordinary skill in the art should understand that those skilled in the art within the technical scope disclosed in the present disclosure may still modify the technical solutions recorded in the foregoing embodiments or easily conceive of changes within the technical scope disclosed in the present disclosure, or make equivalent replacements for some of the technical features therein within the technical scope disclosed in the present disclosure; and these modifications, changes or replacements do not make the essence of the corresponding technical solutions deviate from the spirit and scope of the technical solutions of the embodiments of the present disclosure, and shall fall within the protection scope of the present disclosure. Therefore, the protection scope of the present disclosure shall be subject to the protection scope of the claims.

## Claims

1. A method for generating a CRF form for a rare disease, **characterized in that** the generating method is applied in a form generation system, the form generation system comprises a user terminal and a server, the server is provided with a medical report database and a medical lexicon, and the generating method runs on the server and comprises:
performing, in response to a keyword input by a user at the user terminal, word frequency statistics on each medical report in the medical report database to determine a candidate word with a highest word frequency in each medical report;
constructing, for each medical term in the medical dictionary and each to-be-compared word, a matrix of (m+1)×(n+1) based on a first string length of the medical term and a second string length of the to-be-compared word, where m is the first string length, n is the second string length, and the to-be-compared word comprises the keyword and the candidate word;
initializing a first row of elements in the matrix sequentially with integers from 0 to n and a first column of elements in the matrix sequentially with integers from 0 to m; for other matrix elements Tij in the matrix, determining other matrix elements Tij according to a preset rule, wherein the preset rule comprises: setting, in response to an i-th character of the medical term and a j-th character of the to-be-compared word being identical, the matrix element Tij equal to a matrix element in an upper-left matrix cell adjacent to a matrix cell where the matrix element Tij is located; setting, in response to an i-th character of the medical term and a j-th character of the to-be-compared word being not identical, the matrix element Tij equal to a sum of a minimum matrix element in upper-left, top, and left matrix cells adjacent to a matrix cell where the matrix element Tij is located and a value 1;
calculating, after determining matrix elements in matrix cells of the matrix, a similarity between the medical term and the to-be-compared word using the following formula: $S = 1 - B / A ;$
where B is a matrix element in a lower-right matrix cell of the matrix, and A is the greater of m and n;
after obtaining a first similarity between each candidate word and each medical term and a second similarity between the keyword and each medical term, determining a target medical term corresponding to the first similarity between the candidate word and the medical term that has a minimum difference from a target similarity, wherein the target medical term is a medical term corresponding to a maximum first similarity for each candidate word, and the target similarity is a maximum second similarity; and
constructing a CRF form based on hierarchical headings in a target medical report corresponding to the target medical term and words matching numerical values in text content corresponding to the hierarchical headings.

2. The generating method according to claim 1, wherein the step of constructing the CRF form based on the hierarchical headings in the target medical report corresponding to the target medical term and the words matching the numerical values in the text content corresponding to the hierarchical headings comprises:
performing, after obtaining the hierarchical headings in the target medical report, tokenization on the hierarchical headings to obtain candidate tokens comprised in the hierarchical headings;
determining, for each candidate token after determining part-of-speech of the candidate token, a distribution ratio of the part-of-speech in various parts-of-speech in the medical field as a weight of the candidate token;
calculating a similarity between the candidate token and a preset medical field term using the following formula: $S = \frac{K . L}{\left‖K\right‖ . \left‖L\right‖} d ,$
where K is a first word vector corresponding to the candidate token, L is a second word vector corresponding to the preset medical field term, **||K||** is a magnitude of the first word vector, ∥L∥ is a magnitude of the second word vector, and d is the weight;
processing text content corresponding to each hierarchical heading to obtain text tokens and a dependency relationship between the text tokens;
determining candidate text tokens that have a dependency relationship with text tokens whose part-of-speech is numeral based on the parts-of-speech of the text tokens;
selecting text tokens having a specified part-of-speech from the candidate text tokens as target text tokens; and
taking candidate tokens with a similarity higher than a preset threshold as parent items, and constructing, for each parent item, the CRF form by taking a target text token comprised in a text content under a heading corresponding to the parent item as a child item.

3. The generating method according to claim 1, further comprising:
displaying the CRF form on the user terminal.

4. The generating method according to claim 1, wherein the user terminal comprises a mobile terminal and a fixed terminal.

5. The generating method according to claim 1, further comprising:
adding, in response to an adding operation of a user, an item corresponding to the adding operation at a position specified by the adding operation.

6. A device for generating a CRF form for a rare disease, wherein the generating device is applied in a form generation system, the form generation system comprises a user terminal and a server, the server is provided with a medical report database and a medical lexicon, and the generating device is configured on the server and comprises:
a statistical unit (31), configured to, in response to a keyword input by a user at the user terminal, perform word frequency statistics on each medical report in the medical report database to determine a candidate word with a highest word frequency in each medical report;
a construction unit (32), configured to, for each medical term in the medical dictionary and each to-be-compared word, construct a matrix of (m+1)×(n+1) based on a first string length of the medical term and a second string length of the to-be-compared word, wherein m is the first string length, n is the second string length, and the to-be-compared word comprises the keyword and the candidate word;
a first determination unit (33), configured to initialize a first row of elements in the matrix sequentially with integers from 0 to n and a first column of elements in the matrix sequentially with integers from 0 to m; for other matrix elements Tij in the matrix, determine other matrix elements Tij according to a preset rule, wherein the preset rule comprises: if an i-th character of the medical term and a j-th character of the to-be-compared word are identical, set the matrix element Tij equal to a matrix element in an upper-left matrix cell adjacent to a matrix cell where the matrix element Tij is located; if an i-th character of the medical term and a j-th character of the to-be-compared word are not identical, set the matrix element Tij equal to a sum of a minimum matrix element in upper-left, top, and left matrix cells adjacent to a matrix cell where the matrix element Tij is located and a value 1;
a calculation unit (34), configured to, after determining matrix elements in matrix cells of the matrix, calculate a similarity between the medical term and the to-be-compared word using the following formula: $S = 1 - B / A ;$
wherein B is a matrix element in a lower-right matrix cell of the matrix, and A is the greater of m and n;
a second determination unit (35), after obtaining a first similarity between each candidate word and each medical term and a second similarity between the keyword and each medical term, determine a target medical term corresponding to the first similarity between the candidate word and the medical term that has a minimum difference from a target similarity, wherein the target medical term is a medical term corresponding to a maximum first similarity for each candidate word, and the target similarity is a maximum second similarity; and
a generation unit (36), configured to construct a CRF form based on hierarchical headings in a target medical report corresponding to the target medical term and words matching numerical values in text content corresponding to the hierarchical headings.

7. The generating device according to claim 6, wherein the generation unit being configured to construct the CRF form based on the hierarchical headings in the target medical report corresponding to the target medical term and the words matching the numerical values in the text content corresponding to the hierarchical headings comprises:
performing, after obtaining the hierarchical headings in the target medical report, tokenization on the hierarchical headings to obtain candidate tokens comprised in the hierarchical headings;
determining, for each candidate token after determining part-of-speech of the candidate token, a distribution ratio of the part-of-speech in various parts-of-speech in the medical field as a weight of the candidate token;
calculating a similarity between the candidate token and a preset medical field term using the following formula: $S = \frac{K . L}{\left‖K\right‖ . \left‖L\right‖} d ,$
where K is a first word vector corresponding to the candidate token, L is a second word vector corresponding to the preset medical field term, ∥K∥ is a magnitude of the first word vector, ∥L∥ is a magnitude of the second word vector, and d is the weight;
processing text content corresponding to each hierarchical heading to obtain text tokens and a dependency relationship between the text tokens;
determining candidate text tokens that have a dependency relationship with text tokens whose part-of-speech is numeral based on the parts-of-speech of the text tokens;
selecting text tokens having a specified part-of-speech from the candidate text tokens as target text tokens; and
taking candidate tokens with a similarity higher than a preset threshold as parent items, and for each parent item, constructing the CRF form by taking a target text token comprised in a text content under a heading corresponding to the parent item as a child item.

8. The generating device according to claim 6, further comprising:
a display unit, configured to display the CRF form on the user terminal.

9. The generating device according to claim 6, wherein the user terminal comprises a mobile terminal and a fixed terminal.

10. The generating device according to claim 6, further comprising:
an adding unit, configured to, in response to an adding operation of a user, add an item corresponding to the adding operation at a position specified by the adding operation.
